## Europäisches Patentamt

## European Patent Office

## Office européen des brevets

(11) Publication number: **0 209 070**
A2

# EUROPEAN PATENT APPLICATION

(21) Application number: 86109358.1

(22) Date of filing: 09.07.86

(51) Int. Cl.⁴: **A 61 M 1/10**

(30) Priority: 15.07.85 US 755107

(43) Date of publication of application: 21.01.87 Bulletin 87/4

(84) Designated Contracting States: BE CH DE FR GB IT LI LU NL SE

(71) Applicant: **Abiomed Cardiovascular Inc., 33 Cherry Hill Road, Danvers Massachusetts 01923 (US)**

(72) Inventor: **Singh, Param I., 40 North Hancock Street, Lexington, MA 02173 (US)**

(74) Representative: **Endlich, Fritz, Dipl.-Phys., Postfach 1326 Blumenstrasse 8, D-8034 Germering (DE)**

(54) **High frequency intra-arterial cardiac support system.**

(57)   Disclosed is a high-frequency intra-arterial cardiac support system having a balloon pump (20) which may be positioned in a major artery downstream of a natural heart. The balloon pump comprises a pumping balloon (14), of small displacement, mounted upon and cyclically inflatable and deflatable by fluid flow through catheter (16) having a lumen leading to the outside of the body. The balloon pump further comprises a valve (15) mounted downstream of said pumping balloon. The system further comprises a control and drive mechanism (18) for providing cyclical fluid flow to said lumen of said catheter for inflation and deflation of said pumping balloon. The cyclical flow and the cyclical inflation and deflation occur at a frequency which is at least three times the normal beating frequency of the natural heart. The small displacement of the pumping balloon is much smaller than the normal stroke volume of the natural heart.

HIGH-FREQUENCY INTRA-ARTERIAL CARDIAC SUPPORT SYSTEM

BACKGROUND OF THE INVENTION

1. Field of the Invention

This invention relates generally to temporary cardiac assist devices used to assist the operation of a failing, traumatized or infarcted heart for a limited period until either the heart recovers or more definitive treatment can be provided. In particular, it relates to so-called intra-aortic balloon pumps. Such a pump does not require major thoracic surgery to connect it to the circulation but is a collapsible structure which may be introduced into an easily accessible large artery, such as a femoral, and may then be guided into some portion of the aorta, usually the thoracic aorta, where it can be employed to assist the left side of the heart, the side most frequently in need of assistance and the driving force behind the systemic circulation. In the usual "counterpulsation" mode of employment, the balloon is pneumatically inflated during diastole to increase blood pressure and deflated during systole to lower the pressure load upon the ventricle. This device and its mode of operation was described in a paper by Moulopolous, Topaz and Kolff, "Diastolic Balloon Pumping in the Aorta - A Mechanical Assistance to the Failing Circulation", American Heart Journal (1962) 63, p.669.

2. Prior Art Problem

Since intra-aortic balloon pumps can be applied with relatively minor surgery and fairly standard vascular catheterization procedures, and afford some useful assistance to the left heart, they are well regarded. However, they provide much less pumping assistance than one would desire, for at least three reasons: first, the conveniently available volume within the aorta is not large compared to the desired stroke volume of the

1

heart; second, the necessity to avoid occlusion of important arteries such as the carotids and renals, and erosion of atherosclerotic plaques, further limits the size of a pump balloon; and third, the elastic compliance of the aortic walls makes the effective pumping displacement of the balloon less than its geometric displacement. Many inventions have been addressed to the alleviation of this problem: For example, U.S. Patent No. 3,504,662 to R.T. Jones shows how to attain better pump fluid dynamics, and U.S. Patent No. 3,692,018 to Goetz and Goetz shows how to direct the limited available flow predominantly to the critical brain and heart circulations. Still, there is no complete solution to the problem of adequate pump output. Also, since the counterpulsation mode requires synchronization with the natural heart, there is a further problem when the available ECG signal is weak, uneven or missing, as may occur in ill patients.

## SUMMARY OF THE INVENTION

It is therefore the principal object of this invention to provide an intra-arterial cardiac support system which can fully support the left heart by pumping the entire stroke volume of the heart while maintaining a low systolic intraventricular pressure. It is a further object of this invention to provide such full support with a balloon pump structure which is physically small, as required for easy insertion and safe operation. Further objects of this invention are to provide means for similar support to the right heart and to provide means for supporting circulation even when the heart is atonic or fibrillating.

According to this invention, the desired objects are attained by positioning a small balloon pump, provided with valve means on its downstream side, in a major artery immediately adjacent to the heart, such as in the ascending aorta between the aortic

2

valve and the ostium of the innominate artery, or in the pulmonary trunk. This small balloon pump is cyclically inflated and deflated at a frequency much higher than that of the beating of the heart, whereby the ejected stroke volume of the ventricle is pumped away by several rapid cycles of balloon pumping. Other objects of this invention, as well as the means for attaining them, are set forth in the accompanying Specification and Drawings, wherein:

### BRIEF DESCRIPTION OF THE DRAWINGS

FIG. 1 is a schematic view of the high-frequency intra-arterial cardiac support system in its mode of employment in support of the left heart.

FIG. 2 is a cross-section view of a preferred embodiment of the balloon pump of this invention as used in the mode of employment illustrated in Figure 1.

FIG. 3 is a schematic view of the high-frequency intra-arterial cardiac support system in its mode of employment in support of the right heart.

FIG. 4 is a cross-section view of a preferred embodiment of the balloon pump of this invention as used in the mode of employment illustrated in Figure 3.

FIG. 5 is a timing diagram illustrating the sequences of inflation and deflation of the parts of the preferred embodiment of the balloon pump.

FIG. 6 is a perspective view of an alternative form of the valve part of the balloon pump.

FIG. 7 is a sagittal cross-section of the valve shown in Figure 6, in the plane indicated by 7--7 in that Figure.

FIG. 8 is a normal cross-section of the valve shown in Figure 6, in the plane indicated by 8--8 in that Figure.

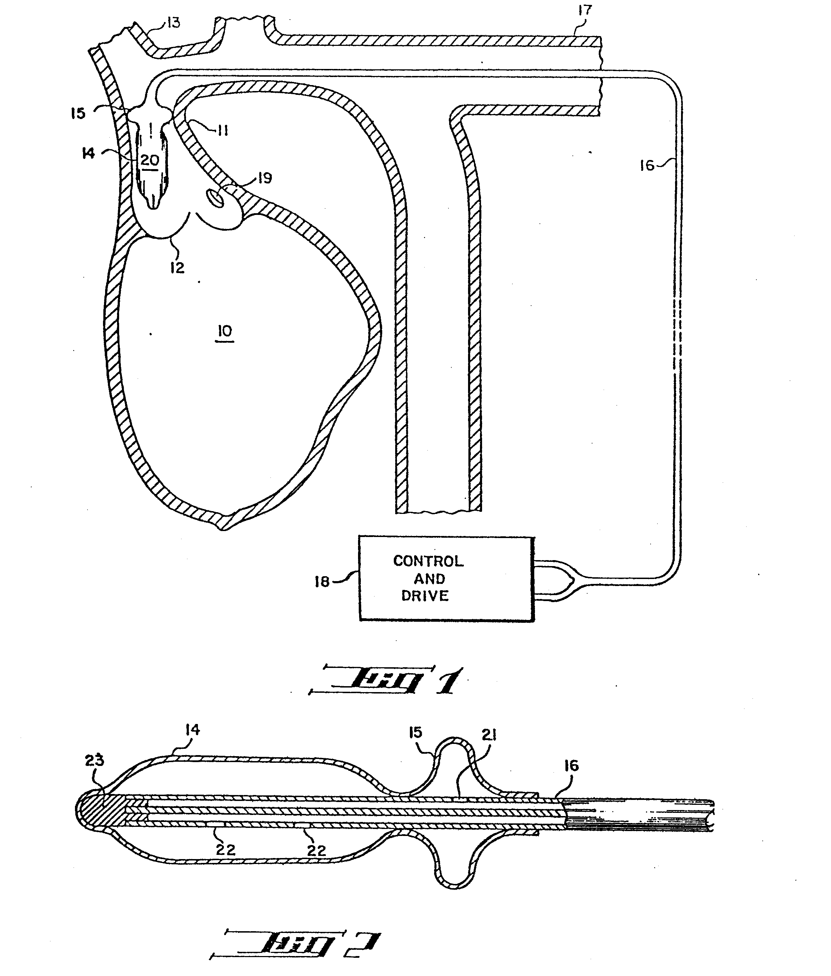

Reference is made to Figure 1, which is a schematic view of the high-frequency intra-arterial cardiac support system in its mode of employment in support of the left heart. Left ventricle 10 is assisted by balloon pump 20 located in ascending aorta 11 between aortic valve 12 and the ostium of innominate artery 13. Pump 20 comprises pumping balloon 14 and valve 15 downstream from balloon 14, both attached to two-lumen catheter 16 which is brought outside the body through the arterial tree, as via subclavian artery 17 in the Figure.

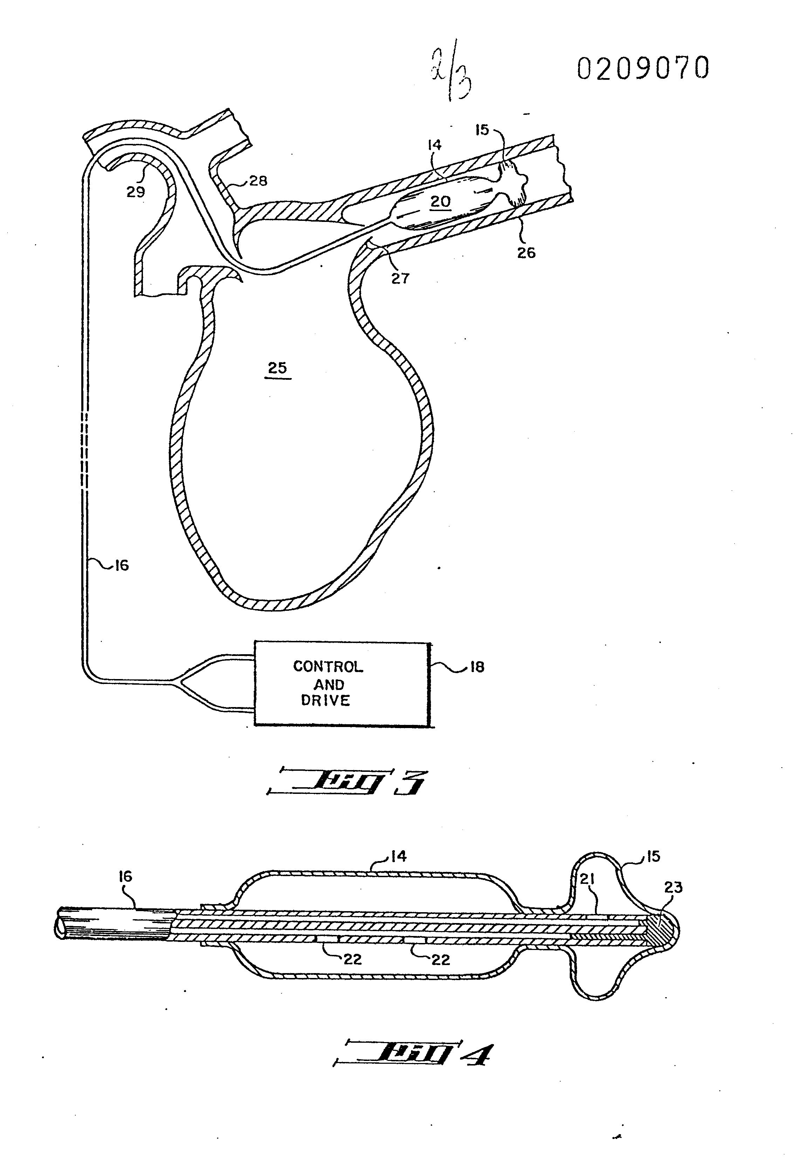

Pump 20, as seen more particularly in Figure 2, comprises a shaped hollow tubular body of thin elastomeric material cemented to two-lumen catheter 16 to form pumping balloon 14 and valve 15. The end of catheter 16 is closed, and the end of balloon 14 is supported, by closure 23, which may advantageously be made of radio-opaque material to facilitate placement of the device. The interior of pumping balloon 14 is connected fluidically to one lumen of catheter 16 by holes 22, and the interior of valve 15 is connected fluidically to the other lumen by hole 21. In the Drawings, dimensions such as thicknesses have been exaggerated in the interest of clarity.

Referring again to Figure 1, it is seen that the outside diameter of valve 15, when erected by inflation, should be just sufficient to substantially occlude ascending aorta 11, and the outside diameter of pumping balloon 14 should be somewhat less. Clearly, a number of sizes will be needed to fit the population of potential patients. In most adults, the inside diameter of the ascending aorta is of the order of of 2.5 centimeters, and the available length for the balloon pump, between the aortic valve and the ostium of the innominate artery, is of the order of 5.0 centimeters.

4

It can also be seen that two-lumen catheter 16 is connected to control and drive mechanism 18 which cyclically and individually inflates and deflates balloon 14 and erects and collapses valve 15 by fluid flow through lumens of catheter 16. The elements of such mechanisms are well known in the art, but the operating parameters of the mechanism of this invention are quite unusual. In particular, the frequencies of inflation and deflation are much higher, and the inflation and deflation volumes are much lower, than those of prior art devices, as will be set forth in greater detail hereinbelow.

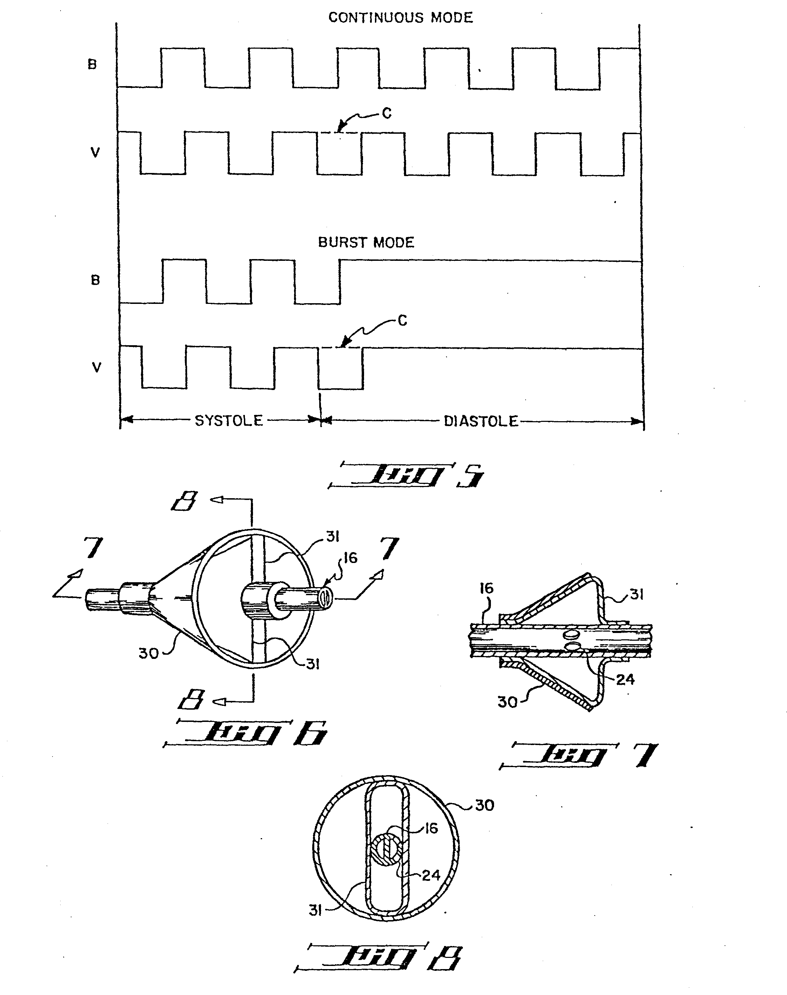

Reference is now made to Figure 3, which is a schematic view of the high-frequency intra-arterial cardiac support system of this invention in its mode of employment in support of the right heart. Here, right ventricle 25 is assisted by balloon pump 20 located in pulmonary trunk 26 just downstream from pulmonary valve 27. In this application, it is preferable that the catheter be placed in the venous system, leading out through right ventricle 25 and superior vena cava 28 and a brachiocephalic vein 29. It will be noted that valve 15 is again located downstream from pumping balloon 14, but that the different direction of approach of the catheter requires that the valve now be placed at the far end of the assembly. This arrangement is shown in detail in Figure 4, where similar numbers refer to similar elements in the arrangement shown in Figure 2.

Reference is now made to Figure 5, which is a timing diagram illustrating the sequence of inflations and deflations of the pumping balloon and the valve of the preferred embodiment of the balloon pump of this invention. Two modes of operation are shown, called respectively "continuous" and "burst". In each case, the letter "B" refers to the pumping balloon and the letter "V" refers to the valve. Upward displacement of a trace signifies

5

increased pressure, or inflation, and downward displacement signifies decreased pressure, or deflation. It will be recognized that the waveforms shown are illustrative only of timing relationships, and that actual balloon and valve pressure and flow waveforms will be quite rounded, approaching the sinusoidal for high frequencies of inflation and deflation.

Examination of the solid-line "continuous mode" traces of Figure 5, in the context of the arrangements shown in Figures 1 and 3, shows that in each case the phasing of the motions of the pumping balloon and the valve, in the presence of the natural valves of the heart, is such as to cause peristaltic pumping of the blood away from the natural valves, and in the direction of natural flow. Considerations of desired pumping throughput and available space for the balloon pump indicate that the balloon pump should make at least three pumping cycles for each beat of the heart, and preferably more, in order to permit a smaller and more easily inserted and positioned balloon pump. This leads to the rule of thumb that the pumping frequency of the balloon pump must be at least three times the beating frequency of the heart.

If it is desired to unload the natural ventricle completely, one may employ the "burst mode" illustrated in Figure 5, in which all the pumping cycles of the balloon pump are accomplished during the period of natural systole. Clearly, in order to provide at least three pumping cycles of the balloon pump within this shortened period, the pumping frequency of the balloon pump must be correspondingly increased. Defining the systolic duty cycle as being the fraction of the total heartbeat period occupied by the systolic period leads to a rule of thumb for the burst mode: that the pumping frequency of the balloon pump must be at least three times the beating frequency of the heart divided by the systolic duty cycle.

6

If it is desired to increase perfusion of the coronary arteries in a system used to assist the left heart, one may make a slight modification of the control waveforms as indicated by the dashed lines labelled "C" in Figure 5. It can be seen that the effect is to omit a deflation of the valve and momentarily to impede outflow from the pump during inflation of the pumping balloon, and application of a pulse of blood pressure to ostia of the coronary arteries, such as the one shown at 19 in Figure 1. One or more of these events may be used advantageously at the beginning of diastole, as shown for burst mode, or occasionally during continuous mode.

Since pump throughput is essentially proportional to the product of pump displacement and pumping frequency, it follows that higher pumping frequencies permit smaller pumps. At lower pumping frequencies, pump throughput is limited largely by fluid inertia and viscosity forces in the catheter, and is only weakly dependent on pump displacement up to frequencies at which blood inertia effects become dominant; this leads to a preference for the highest practical pumping frequency. Analysis indicates that a pumping frequency of 1000 strokes per minute is attainable, or almost 14 times the nominal adult heart rate, using helium gas as the drive fluid. It is further likely that arterial compliance effects may be less pronounced at such higher frequencies. For one example, a small pumping balloon of only 7.5 ml displacement, at a pumping frequency of 12 per second and a displacement efficiency of 75% may yield a generous 4 liters per minute.

At higher pumping frequencies, the precise phase relationship between the individual pumping cycles and the cardiac cycle is quite immaterial. Therefore, in the continuous mode, synchronization with the EKG is unnecessary. For this reason, when used bilaterally, this system can support circulation even when the heart is atonic or fibrillating.

7

It is not absolutely necessary to use an externally-driven valve in order to accomplish pumping. Indeed, some pumping action can be observed if the valve be simply erected by inflation to some moderate constant pressure, especially if a liquid be used as the inflating fluid. A preferable arrangement is to use a one-way passive check valve, several of which have been suggested in the literature. For this purpose, I prefer the structure shown in Figures 6, 7 and 8 which are, respectively, a perspective view and sagittal and normal cross-sections of my preferred passive valve. As shown in the Figures, it is an umbrella-like structure comprising inflatable ribs 31 supporting a flexible canopy 30, both made of thin elastomeric material. The structure is mounted upon and cemented to two-lumen catheter 16, and the ribs are fluidically connected to one of the lumens by holes 24, so that they can be erected and collapsed by fluid, preferably a liquid, supplied from the outside through a lumen in the catheter.

Given the foregoing teaching, those skilled in the art to which this invention pertains may readily devise further or extended embodiments. For example, in cases where one or more of the valves of the natural heart are damaged or otherwise incompetent, one may provide the balloon pump with an additional valve at the upstream end, either an externally-driven valve as shown in Figures 1 and 3, driven through an additional catheter lumen, or a passive valve as shown in Figures 6, 7 and 8. Various other features and advantages not specifically enumerated will occur to those versed in the art, as likewise many variations of the embodiments which have been illustrated, all of which may be achieved without departing from the spirit and scope of the invention as defined by the following claims:

8

I claim:

1. A high-frequency intra-arterial cardiac support system comprising a balloon pump which may be positioned in a major artery downstream of a natural heart, said balloon pump comprising a pumping balloon, of small displacement, mounted upon and cyclically inflatable and deflatable by fluid flow through catheter having a lumen leading to the outside of the body and said balloon pump further comprising a valve mounted downstream of said pumping balloon, said system further comprising a control and drive mechanism for providing cyclical fluid flow to said lumen of said catheter for inflation and deflation of said pumping balloon, said system being further characterized by:

said cyclical flow and said cyclical inflation and deflation occurring at a frequency which is at least three times the normal beating frequency of the natural heart; and

said small displacement of said pumping balloon being much smaller than the normal stroke volume of the natural heart.

2. A high-frequency intra-arterial cardiac support system according to claim 1 operable in burst mode wherein said cyclical fluid flow occurs mostly during systole of the natural heart said cyclical flow and said cyclical inflation and deflation occurring at a frequency which is at least three times the normal beating frequency of the natural heart, divided by the systolic duty cycle.

3. A high-frequency intra-arterial cardiac support system according to claim 1 in which said fluid flow is flow of a gas.

4. A high-frequency intra-arterial cardiac support system according to claim 1 in which said balloon pump comprises a further valve mounted upstream of said pumping balloon.

5. A high-frequency intra-arterial cardiac support system according to claim 1 in which said valve is a collapsible one-way check valve directing blood flow away from the natural heart, and the diameter of said valve, when erected, is just sufficient to substantially occlude said major artery.

6. A high-frequency intra-arterial cardiac support system according to claim 1 in which said catheter has a further lumen, said valve is a small balloon of inflated diameter just sufficient to substantially occlude said major artery and erectable and collapsible by inflation and deflation through said further lumen, and said control and drive mechanism provides a further cyclical fluid flow to said further lumen to operate said valve by cyclical inflation and deflation in suitable phase with said pumping balloon to cause pumping of blood away from the natural heart.

7. A high-frequency intra-arterial cardiac support system according to claim 6 and configured for support to the left heart by a balloon pump positioned in the ascending aorta, in which occasional deflations of said valve are omitted.

8. A high-frequency intra-arterial cardiac support system according to claim 7, operable in burst mode with said cyclical fluid flows occurring mostly during systole of the natural heart, in which said omitted deflations of said valve occur near the beginning of diastole of the natural heart.

10

9. For use in a high-frequency intra-arterial cardiac support system, a balloon pump, for positioning in a major artery downstream of a natural heart and comprising an erectable valve contiguous to the downstream side of a pumping balloon mounted upon a catheter leading to the outside of the body, said pumping balloon being cyclically inflated and deflated through a lumen of said catheter at a frequency at least three times the normal beating frequency of the natural heart, said valve having an erected diameter just sufficient to substantially occlude said major artery, and said pumping balloon having an inflated diameter which is less than said erected diameter.

10. A balloon pump according to claim 9, for providing support to the left heart and for positioning in the ascending aorta between the aortic valve and the ostium of the innominate artery, wherein said pumping balloon is mounted at the end of said catheter and coaxial therewith, said valve is mounted contiguous to said pumping balloon upon said catheter and coaxial therewith, and the total length of said pumping balloon and said valve, parallel to said catheter, is not greater than the distance between said aortic valve and said ostium.

11. A balloon pump according to claim 9, for providing support to the right heart and for positioning in the pulmonary trunk, wherein said valve is mounted upon the end of said catheter and coaxial therewith, said pumping balloon is mounted contiguous to said valve upon said catheter and coaxial therewith, and the total length of said valve and said pumping balloon, parallel to said catheter, is not greater that the length of said pulmonary trunk.

11

1/3

CONTROL
AND
DRIVE

_Fig 1_

_Fig 2_

**CONTROL AND DRIVE**

_Fig 3_

_Fig 4_

CONTINUOUS MODE

B

V                    C

BURST MODE

B

V                    C

SYSTOLE — DIASTOLE

_Fig 5_

_Fig 6_

_Fig 7_

_Fig 8_